# EUROPEAN PATENT SPECIFICATION

(11) **EP 0 631 784 B1**
(45) Date of publication and mention of the grant of the patent: **31.03.1999**
(21) Application number: 94103441.5
(22) Date of filing: 22.01.1988
(51) Int. Cl.: A61K 31/725

(54) **Sulphated polysaccharides having anti-inflammatory activity**
Sulfatierte Polysaccharide mit antiinflammatorischer Aktivität
Polysaccharides sulphatés ayant une activité antiinflammatoire

(30) Priority: 23.01.1987 AU 9991/87
(43) Date of publication of application: 04.01.1995
(62) Divisional of application: 88901519.4
(73) Proprietor: THE AUSTRALIAN NATIONAL UNIVERSITY, Acton, Australian Capital Territory 2601 (AU)
(72) Inventor: Parish, Christopher Richard, Macquarie, 2614 A.C.T. (AU); Snowden, John McKinnon, Leeming, 6155 W.A. (AU)
(74) Representative: Woods, Geoffrey Corlett

(56) References cited:
- EP-A- 0 251 134
- WO-A-88/07060
- THERAPIE, 1973, VOL. 28, PAGE(S) 889-905 Giroud, J. P. et al 'Relations between antiinflammatory and anticomplement activity of some sulfated polysaccharides having anticoagulant properties'
- WISS. Z. - MARTIN-LUTHER-UNIV. HALLE-WITTENBERG, MATH.-NATURWISS. REIHE, 1978, VOL. 27, PAGE(S) 35-49 Hirschelmann, Rolf et al 'Experimental comparison of the antiinflammatory effect of cytostatics, peptides, proteins, protease inhibitors, neuroleptics, narcotic and non-narcotic analgesics, adrenaline, histamine-serotonin antagonists and antiinflammatory/antirheumatic drugs'
- ARCH NEUROL (UNITED STATES), AUG 1972, VOL. 27, NO. 2, PAGE(S) 153-8, Chelmicka-Szorc E et al 'Partial suppression of experimental allergic encephalomyelitis with heparin.'
- WIEN. KLIN. WOCHENSCHR., 1978, VOL. 90, PAGE(S) 101-5 Kalbhen, D. A. 'Biochemical and pharmacological basis of the antiphlogistic/antirheumatic effect of pentosan polysulfate'
- THROMB HAEMOST (GERMANY, WEST), OCT 31 1984, VOL. 52, NO. 2, PAGE(S) 134-7, Matzner Y et al 'The inhibitory effect of heparin and related glycosaminoglycans on neutrophil chemotaxis.'
- CHEMICAL ABSTRACTS, vol. 105, no. 2, 14 July 1986, Columbus, Ohio, US; abstract no. 12086g, U. OSAMU ET AL. 'Preparation of fucoidan from seaweed' page 372 ;column 1 ; & JP-A-6 157 520 (...)
- SYMPOSIA CONFERENCES HELD BY THE BRITISH SOCIETY FOR CELL BIOLOGY IN CONJUNCTION WITH THE BRITISH SOCIETY FOR DEVELOPMENTAL BIOLOGY, OXFORD, ENGLAND, UK, MARCH,, VOL. 11, NO. 3, PAGE(S) 253, 1987. COOMBE D R et al 'SULFATED POLYSACCHARIDES WITH ANTIMETASTATIC ACTIVITY ACT BY INHIBITING TUMOR CELL SECRETED ENDOGLYCOSIDASES'
- BIOCHEMISTRY, vol.25, 1986 pages 5322 - 5328 T. IRIMURA ET AL. 'Chemically modified heparins as inhibitors of heparan sulfate specific endo-beta-glucuronidase (Heparanase) of metastatic melanoma cells'
- J IMMUNOL (UNITED STATES), MAY 15 1988, VOL. 140, NO. 10, PAGE(S) 3401-5, WILLENBORG DO ET AL 'Inhibition of allergic encephalomyelitis in rats by treatment with sulfated polysaccharides.'

## Description

This invention relates to compounds having anti-inflammatory activity, and in particular it relates to the use of these compounds as anti-inflammatory agents in animals and man.

One of the key events in inflammation is the adherence of leukocytes or tumour cells to blood vessel walls and their subsequent emigration into tissues. The molecular basis of these processes is poorly understood although it is generally accepted that penetration of the vessel wall requires localised degradation of the interendothelial cell junctions and subendothelial matrix by specific degradative enzymes.

It has now been discovered that continuous infusion of certain sulphated polysaccharides, such as heparin and fucoidan, can completely prevent experimental allergic encephalmyelitis (EAE), an animal inflammatory disease similar to multiple sclerosis in humans.

In a first aspect, the present invention relates to the use in the manufacture of a medicament for the anti-inflammatory treatment of an animal or human patient of at least one sulphated polysaccharide which blocks or inhibits endoglycosidase activity, said sulphated polysaccharide being fucoidan or periodate-oxidised, reduced heparin.

In another aspect, this invention relates to a pharmaceutical or vetinary composition for anti-inflammatory treatment which comprises at least one sulphated polysaccharide which blocks or inhibits endoglycosidase activity, together with a pharmaceutically acceptable carrier or diluent therefor, said sulphated polysaccharide being fucoidan or periodate-oxidised, reduced heparin.

This invention particularly relates to the use of sulphated polysaccharides which block heparinase activity. One particularly preferred active component is heparin that has been appropriately modified to reduce anti-coagulant activity, namely periodate oxidized, reduced heparin that has negligible anti-coagulant activity but is a potent anti-metastatic agent. The endoglycosidase-inhibitory activity of the polysaccharide is retained.

The following Example demonstrates the anti-inflammatory activity of the sulphated polysaccharides, modified heparin and fucoidan.

### EXAMPLE 1

### MATERIALS AND METHODS

Rats. Lewis (RT-1¹) rats were bred at the John Curtin School of Medical Research. Both males and females of 8-10 weeks of age were used. In each experiment controls and experimental rats were matched for sex.

### Induction of EAE.

Active. Guinea pig BP was prepared according to the method of Deibler et al ( 2 ) and BP in saline was emulsified in an equal volume of incomplete Freund's adjuvant containing 4 mg/ml added Mycobacterium butyricum. Rats received 0.1 ml emulsion in one footpad of both hind feet. Total dose received was 50 µg BP and 400 µg Mycobacterium butyricum.

Passive. Cells for passive transfer were generated following the method of Panitch and McFarlin ( 3 ) Single cell suspensions were prepared from spleens of donor rats sensitized 12 days previously with BP-CFA as described above. Cells were cultured at 2x10⁶/ml in RPMI 1640 +5% fetal calf serum, 5x10⁻⁵M 2-mercaptoethanol, 200 mM L-glutamine and penicillin and streptomycin. Con A was added at 2 µg/ml and cultures were incubated at 37°C in an atmosphere of 10% CO₂, 7% O₂ and the balance N₂. Cells were harvested after 72 hrs, washed with Hanks balanced salt solution (BSS) and transferred to recipients via a lateral tail vein. All transfer populations contained 30x10⁶ viable cells.

### Evaluation of clinical EAE

Clinical EAE was graded according to the following scheme-: 0, asymptomatic; 1, flaccid distal half of tail; 2, entire tail flaccid; 3, ataxia, difficulty in righting; 4, hind limb weakness; 5, hind limb paralysis.

In most experiments we also calculated the mean day of onset of disease (MDO), the mean clinical score (MCS) and the mean length or duration of disease (MLD). Values are expressed standard error of the mean.

### Histology

Rats were perfused with 10% neutral buffered formal in. Their spinal cords removed and prepared by standard histological techniques. Slides were stained with H and E.

### Polysaccharides

Fucoidan (from Fucus vesiculosus) and heparin (sodium salt from bovine lung) were, and also chondroitin-4-sulphate and pentosan polysulphate for comparative experiments, purchased from Sigma (St. Louis, MO). The polysaccharides were dissolved in 0.15 M NaCl and stored at -20°C. They were thawed and then boiled for 2 min immediately before use.

Heparin devoid of anticoagulant activity was prepared by periodate oxidation and borohydrate reduction using a similar procedure to that described by Fransson ( 1 ). Bovine lung heparin (10 mg/ml) was dissolved in 0.05 M sodium phosphate buffer, pH 7.0, containing 40 mM sodium periodate and left to react for 18 hrs at 37°C in the dark. The reaction was stopped by the addition of solid D-mannitol (5 mg/ml) and the solution then dialyzed against distilled water at room temperature for 2 hrs, the dialysate being changed every 30 min. The oxyheparin was then reduced by the addition of solid potassium borohydride (2 mg/mg heparin), the reduction reaction being left for 3 hrs at room temperature and then terminated by the addition of glacial acetic acid (1 µl/mg of borohydride). The heparin was then ethanol precipitated twice (2 vols ethanol, 4°C, 18 hrs) and finally dissolved in 0.15 M NaCl to a concentration of 20 mg/ml. Approximately 50% of the heparin was recovered as the periodate oxidised borohydride reduced preparation.

The anticoagulant activity of heparin and periodate oxidized heparin was determined using rat plasma in the activated partial thromboplastin time and thrombin time tests . Based on these assays periodate oxidation resulted in a 500-2000 fold reduction in the anticoagulant activity of heparin.

Delivery of sulfated polysaccharides. Because of the short half life of some of the polysaccharides in vivo we thought it necessary to give repeated doses. We first attempted ip injections of heparin every twelve hours. Unfortunately, this produced unacceptable levels of hemorrhage and even death of some animals. We chose therefore to use mini osmotic pumps (type 2ML, ALZA Corp, Palo Alto, Calif.) which were implanted subcutaneously in the back. The pumps have a 2ml capacity and deliver approximately 10 µl/hr for 7 days. Plasma levels of heparin was measured in rats implanted with an osmotic pump containing 20 mg/ml. The method employed was essentially the dimethylmethylene blue procedure of Farndale et al ( 4 ). A steady state concentration of 10-20 µg/ml was reached by 24 hrs after implanting the pump on day 0 and no heparin was detectable on day 8, i.e. 24 hrs after the pump ceased to deliver.

### RESULTS

Recipient rats received 30x10⁶ EAE effector cells and at the same time osmotic pumps were placed subcutaneously in the back. The pumps contained 2 ml of heparin at either 10 mg/ml or 20 mg/ml. As shown in Table I there was a degree of protection in both heparin treated groups. Only 3 of 6 rats receiving 10 mg/ml heparin developed disease and only 1 of 5 receiving 20 mg/ml. In the latter case, the one animal which did develop disease did so later after cell transfer and also exhibited a milder disease.

In the next experiment we used heparin again as well as three further sulfated polysaccharides, fucoidan, chondroitin-4-sulfate, and pentosan sulfate, and also asked if initiation of treatment could be delayed for 3 days and still provide protection. Fucoidan as well as heparin gave complete protection against EAE even when treatment was delayed till day 3 after cell transfer (Table II). Pentosan sulfate gave partial protection as evidenced by later onset, milder clinical disease, and shorter duration of disease. Chondroitin-4-sulfate had no protective effect.

Often, therapeutic studies of various agents in EAE demonstrate that clinical disease may be abrogated, yet histopathologic examination will reveal quite extensive inflammatory lesions (5, 6 ). To examine this in the present context, 3 control and 3 heparin treated animals from the experiment described in Table II were killed on day 8 post cell transfer and examined for inflammatory lesions. Virtually every low power field of a 2 cm longitudinal section through the lower thoracic/upper lumbar cord of control rats had numerous perivascular inflammatory lesions.

In contrast, no lesions were seen in any of 80 sections from the same area of the three heparin treated rats.

To determine if the sulfated polysaccharides are Inhibiting adoptive EAE by simply killing the transferred cells, we examined the ability of treated rats to exhibit memory to a challenge with BP-CFA. We ( 7, 8) and others ( 9, 10) have reported that following recovery from passively Induced EAE, or in the case of neonates, in the absence of any initial disease symptoms following cell transfer (7, 8), a later active challenge with BP-CFA leads to a much earlier onset of disease symptoms than is seen in control animals which never received EAE effector cells. The interpretation of these data is that early onset is the result of activation of memory cells which persist in the animal and arise from the original transfer population. Therefore, the animals represented in Table II were challenged with 50 g BP-CFA on day 14 after receiving the cell transfer. Control rats which had never received cells were also challenged. The results are shown in Table III Naive animals developed disease day 10-11 after active immunization. Cell recipients on the other hand showed a memory response following challenge irrespective of treatment regimen or presence or absence of initial clinical signs, thus demonstrating that treatment did not inhibit adoptively transferred disease by killing the cells.

Is the effect that heparin, and possibly the other polysaccharides have on adoptively transferred EAE a function of their anticoagulant activity? To answer this question, anticoagulant free heparin was prepared by periodate oxidation and borohydride reduction as described above and then tested for its EAE inhibiting activity. As seen in Table IV, all animals receiving anticoagulant free heparin developed EAE; however, there was a significant delay in onset of disease, a diminution in the clinical severity and also a decrease in the duration of the disease symptoms. These results strongly suggest that the EAE inhibiting effect of heparin is not due solely to its anticoagulating activity.

The effect of heparin on actively induced EAE was also examined and the results presented in Table v. When heparin treatment was begun at the time of sensitization there was a sgnificant delay in the onset of disease, however, the clinical score attained or the duration of disease did not differ from controls animals. It is interesting to note that the delay of 6 days is approximately the length of time the pumps deliver heparin, 7 days.

**TABLE I**

| Effect of heparin on adoptively transferred EAE^{a} | | | |
|---|---|---|---|
| Treatment | # Rats with EAE/Total | Mean Day of Onset | Mean Clinical Score |
| Control | 4/4 | 4.3. ±0.2 | 3.9 ±0.1 |
| Heparin^{b} (10 mg/ml) | 3/6 | 5.3 ±0.3^{c} | 3.5 ±0.2^{c} |
| Heparin^{b} (20 mg/ml) | 1/5 | 7.0^{c} | 2.5^{c} |

| | | | |
|---|---|---|---|
| a. 30x10⁶ Con A incubated EAE spleen cells given iv. | | | |
| b. Heparin given in osmotic pumps placed subcutaneously at time of cell transfer. | | | |
| c. Represent the score only for the animal(s) which developed clinical signs. | | | |

**TABLE II**

| Effect of sulfated polysaccharides on adoptively transferred EAE | | | | |
|---|---|---|---|---|
| Treatment | # With EAE/Total | Mean Day Onset | Mean Clinical Score | Mean Length Disease |
| Control | 7/7 | 4.4 ±0.2 | 4.1 ±0.9 | 5.0 ±0.4 |

| Comparative Example | | | | |
|---|---|---|---|---|
| Heparin (20 mg/ml) | 0/8 | - | - | - |

| Example | | | | |
|---|---|---|---|---|
| Fucoidan (20 mg/ml) | 0/6 | - | - | - |

| Comparative Examples | | | | |
|---|---|---|---|---|
| Chondroitin-4-sulfate (20 mg/ml) | 5/5 | 5 | 3.5 ±0.2 | 5.0 ±0.4 |
| Pentosan sulfate (20 mg/ml) | 5/5 | 6.2 ±0.3 | 2.4 ±0.2 | 3.0 ±0.4 |

| | | | | |
|---|---|---|---|---|
| a. Osmotic pumps containing sulfated polysaccharides were implanted day 3 after cell transfer. | | | | |

**TABLE III**

| Memory response in rats receiving sulfated polysaccharides 3 days after adoptive transfer of EAE effector cells | | |
|---|---|---|
| Treatment Group | # With EAE/# Challenged^{a} | Individual Day of Onset |
| Naive | 4/4 | 10, 11, 11, 11 |
| Control cells only) | 4/4 | 7, 7, 7, 8 |

| Comparative Example | | |
|---|---|---|
| Heparin | 5/5 | 7, 7, 7, 8, 8 |

| Example | | |
|---|---|---|
| Fucoidan | 6/6 | 7, 7, 8, 8, 8, 10 |

| Comparative Examples | | |
|---|---|---|
| Chondroitin-4-sulfate | 5/5 | 7, 7, 8, 8, 10 |
| Pentosan sulfate | N.D.^{b} | N.D. |

| | | |
|---|---|---|
| a. Rats Challenged with 50 µg BP-CFA on day 14 after initial cell transfer. | | |
| b. Not determined. | | |

**TABLE IV**

| Effect of anticoagulant-free heparin on passively induced EAE | | | | |
|---|---|---|---|---|
| Treatment | # With EAE/Total | Mean Day Onset | Mean Clinical Score | Mean Duration Disease |
| Control | 7/7 | 5.0 | 3.4 ±0.1 | 4.0 ±0.2 |
| Heparin (20 mg/ml) | 0/5 | - | - | - |

| Example | | | | |
|---|---|---|---|---|
| Periodate-oxidised heparin (20 mg/ml) | 5/5 | 6.4 ±0.2 | 1.6 ±0.2 | 2.4 ±0.4 |

**TABLE V**

| Effect of heparin on actively induced EAE (comparative) | | | | |
|---|---|---|---|---|
| Treatment | # With EAE/# Challenged | Mean Day Onset | Mean Clinical Score | Mean Duration Disease |
| Control | 5/5 | 11.4 ±0.5 | 4.2 ±0.3 | 6.6 ±0.6 |
| Heparin^{a} (20 mg/ml) | 5/5 | 17.6 ±0.8 | 5.0 | 6.0 ±0.5 |

| | | | | |
|---|---|---|---|---|
| a. Heparin pumps implanted at time of sensitization with 50 µg BP-CFA/rat. | | | | |

1. FRANSSON, L-A. Carbohydr. Res. 62, 235-244 (1978).
2. Deibler G.E., R.E. Martinson and M.W. Kies. Prep. Biochem. 2: 139 (1972).
3. Panitch H.S. and D.E. McFarlin. J. Immunol. 119: 1134. (1977).
4. Farndale R.W., D.J. Buttle and A.J. Barrett. Biochem.et.Biphys.Acta. 883: 173. (1986).
5. Welch A., J.H. Holda and R.H. Swanborg. J. Immunol. 125: 186. (1980).
6. Hauser S.L., H.L. Weiner, M. Che, M.F. Shapiro, F. Gilles and N.L. Letvin. J. Immunol. 132: 1276. (1984).
7. Willenborg D.O. and G. Danta. Clin.Exp.Neurol. 21: 225. (1985).
8. Willengborg D.O., P. Sjollema and G. Danta. Scand.J.Immunol. 23: 75. (1986).
9. Holda J.H. and R.H. Swanborg. Immun.Commun. 9: 333. (1980).
10. Hinrichs D.J., C.M. Roberts and F.J. Waxman. J.Immunol. 126: 1857. (1981).

## Claims

1. Use in the manufacture of a medicament for the anti-inflammatory treatment of an animal or human patient of at least one sulphated polysaccharide which blocks or inhibits endoglycosidase activity, said sulphated polysaccharide being fucoidan or periodate-oxidised, reduced heparin.

2. Use according to claim 1 wherein said sulphated polysaccharide is one which blocks or inhibits heparinase activity.

3. Use according to claim 1 wherein said sulphated polysaccharide is heparin which has been modified to reduce its anti-coagulant activity.

4. Use according to claim 3, wherein said modified heparin is periodate oxidized, reduced heparin.

5. A pharmaceutical or veterinary composition for anti-inflammatory treatment which comprises at least one sulphated polysaccharide which blocks or inhibits endoglycosidase activity, together with a pharmaceutically or veterinarily acceptable carrier or diluent therefor, said sulphated polysaccharide being fucoidan or periodate-oxidised, reduced heparin.

6. A composition according to claim 5, wherein said sulphated polysaccharide is one which blocks or inhibits heparanase activity.

7. A composition according to claim 5, wherein said sulphated polysaccharide is fucoidan.

## Patentansprüche

1. Verwendung mindestens eines sulfatierten Polysaccharids, das die Aktivität der Endoglycosidase blockiert oder inhibiert, wobei das sulfatierte Polysaccharid Fucoidan oder durch Perjodat oxidiertes und reduziertes Heparin ist, zur Herstellung eines Arzneimittels zur antiinflammatorischen Behandlung von Tieren oder Menschen als Patienten.

2. Verwendung nach Anspruch 1, worin das sulfatierte Polysaccharid ein solchesist, das die Aktivität der Heparinase blockiert oder inhibiert.

3. Verwendung nach Anspruch 1, worin das sulfatierte Polysaccharid ein Heparin ist, das zur Verringerung seiner Anticoagulanzwirkung modifiziert wurde.

4. Verwendung nach Anspruch 3, worin das modifizierte Heparin ein durch Perjodat oxidiertes und reduziertes Heparin ist.

5. Pharmazeutische oder veterinärmedizinische Zusammensetzung zur antiinflammatorischen Behandlung, die wenigstens ein sulfatiertes Polysaccharid, das die Aktivität der Endoglycosidase blockiert oder inhibiert, und einem pharmazeutisch oder veterinärmedizinisch verträglichen Träger oder verträgliches Verdünnungsmittel dafür enthält, wobei das sulfatierte Polysaccharid Fucoidan oder ein durch Perjodat oxidiertes und reduziertes Heparin ist.

6. Zusammensetzung nach Anspruch 5, worin das sulfatierte Polysaccharid ein solches ist, das die Aktivität der Heparinase blockiert oder inhibiert.

7. Zusammensetzung nach Anspruch 5, worin das sulfatierte Polysaccharid Fucoidan ist.

## Revendications

1. Utilisation dans la fabrication d'un médicament pour le traitement anti-inflammatoire d'un sujet humain ou animal d'au moins un polysaccharide sulfaté qui bloque ou inhibe l'activité endoglycosidase, ledit polysaccharide sulfaté étant du fucoïdane ou de l'héparine oxydée au périodate et réduite.

2. Utilisation selon la revendication 1, dans laquelle ledit polysaccharide est un polysaccharide qui bloque ou inhibe l'activité héparinase.

3. Utilisation selon la revendication 1, dans laquelle ledit polysaccharide sulfaté est de l'héparine ayant été modifiée afin de réduire son activité anticoagulante.

4. Utilisation selon la revendication 3, dans laquelle ladite héparine modifiée est de l'héparine oxydée ou périodate et réduite.

5. Composition pharmaceutique ou vétérinaire pour un traitement anti-inflammatoire, qui comprend au moins un polysaccharide sulfaté qui bloque ou inhibe l'activité endoglycosidase, avec un véhicule ou un diluant acceptable du point de vue pharmaceutique ou vétérinaire, ledit polysaccharide sulfaté étant du fucoïdane ou de l'héparine oxydée au périodate et réduite.

6. Composition selon la revendication 5, dans laquelle ledit polysaccharide sulfaté est un polysaccharide qui bloque ou inhibe l'activité héparinase.

7. Composition selon la revendication 5, dans laquelle ledit polysaccharide sulfaté est le fucoïdane.
